# EUROPEAN PATENT APPLICATION

(11) **EP 4 191 609 A1**
(43) Date of publication of application: **07.06.2023**
(21) Application number: 22211558.6
(22) Date of filing: 06.12.2022
(51) Int. Cl.: G16H 50/20, G16H 50/70

(54) **COMPUTER IMPLEMENTED METHOD FOR ANALYZING MEDICAL DATA, SYSTEM FOR ANALYZING MEDICAL DATA AND COMPUTER READABLE MEDIUM STORING SOFTWARE**

(30) Priority: 06.12.2021 EP 21212583
(71) Applicant: Zurich Insurance Company Ltd., 8002 Zürich (CH)
(72) Inventor: Lacroix, Sibylle, 8808 Pfäffikon (CH); Nadworny, Bartosz, 8037 Zürich (CH); Borgschulte, Philipp, 8600 Dübendorf (CH)
(74) Representative: Hepp Wenger Ryffel AG

(57) **Abstract**

A computer implemented method for analyzing medical data is disclosed that allows for analyzing (a) medical data record(s) of interest by comparing at least one medical data record of interest to a benchmark set selected from clustered data elements; and/or predicting risks associated with a medical data record of interest or a set of medical data records of interest based on classification models, preferably trained by supervised machine learning.

## Description

The invention pertains to the technical field of information technology adapted for data mining and risk recognition based on a set of previous medical data records.

The volume of data accumulated by institutions and individuals in the healthcare domain grows exponentially. By mining large amounts of data, correlations, factors and effects may be discovered and corresponding risks detected. Thanks to language recognition and processing tools, the systematic analysis of extensive text records has in principle become available for statistical analysis. The problem with large data sets related to a plurality of patients, however, is that the amount of time required to process them is excessive, thus hindering a rapid analysis in practice. If this drawback is remedied by applying traditional compressing mechanisms, the loss of information may be such that commonalities between cases are no longer detectable or detectable only to an insufficient extent.

Systems for the processing and presenting data related to a patient have been described in the art, for example in EP3223183. This document aims at extracting a plurality of different medical data sets of different dimensionalities (e.g., images and text files), generating data signatures which harmonize the dimensionality of the data sets and combining the generated data signatures to a joint data signature based thereon.

However, there remains a need in the art to process a large number of documents of a certain diversity and related to a plurality of patients in a fast and sufficiently detailed manner.

It is the purpose of the present invention to overcome the disadvantages in the state of the art. In particular, it is the purpose of the present invention to suggest a method which allows to retrieve an amount of data and metadata from a set of previous medical data records for analysis purposes, wherein the processing of data ensures a proper balance between calculation speed and the goal to preserve as much relevant information as possible.

The problem has been solved by means of a method for analyzing medical data, a system and a storage medium according to the independent claims.

The computer implemented method for analyzing medical data according to the invention comprises the steps of:
a) providing a natural language processing asset (NLP), wherein the NLP includes a graph-based ontology of medical terms derived from a medical knowledge domain;
b) providing a set of previous medical data records, in particular diagnostic medical data records and/or therapeutic medical data records;
c) extracting and annotating a plurality of data elements from the set of previous medical data records, wherein the extracting and annotating is based on
   - the NLP provided in step a), and
   - classification models trained by supervised machine learning;
d) matching extracted and annotated data elements of step c) to the medical terms retrieved in step a) and assigning the matched data elements to the graph based ontology;
e) enriching matched the data elements of step d)
   - by associating a first data element with at least a second data element sharing a parent with the first data element within the ontology, and/or
   - by assigning a first data element to a subordinate or superordinate level of the ontology;
f) clustering the enriched data elements of step e) based on concepts retrieved from the graph based ontology;
g) analyzing a medical data record of interest or a set of medical data records of interest by
   - comparing at least one medical data record of interest, preferably a set of medical data records of interest, to a benchmark set selected from the clustered data elements, and/or
   - predicting risks associated with a medical data record of interest or a set of medical data records of interest based on classification models, preferably trained by supervised machine learning; wherein the risks are medical risks.

The invention is based on the idea that a series of documents is usually accumulated in connection with an individual medical case, such as medical reports, clinical notes, discharge summaries, clinical trial protocols, laboratory test results, clinical and family history, medications, medical questionnaires, health surveys, admission reports, healthcare app data, diagnoses, radiology documents, physiologic monitoring data, clinical research datasets etc; hereinafter collectively referred to as medical data records or simply data records. In order to exploratively analyze such data records, a large number of cases should ideally be provided for statistical analysis. However, the larger the data set is, the more time is required to consistently process the information by standard software solutions. This applies particularly to text files where a word level comparison needs to be performed. Also, the documents of a medical case are diverse and have different levels of specificity which makes the search for commonalities complex. As a result, associations made by standard software solutions have turned out to be unsuitable for analytical purposes.

The inventors have found a solution to compress data pertaining to a set of medical documents to a processable size, while preserving as much information as necessary to correctly classify the information and to ensure comparability of cases. In a first compression step, data are extracted and annotated on a reduced level of detail. In order to avoid that annotation sets become too specific to find cross case commonalities, the compressed data elements are subsequently re-enriched by means of synonyms in a wider sense and/or by association with terms of a more general and/or more specific level (step e)).

The enriching step e) is enabled by preceding method step d), i.e. by matching the extracted and annotated data elements to the medical terms retrieved in step a) and by assigning the matched data elements to the graph-based ontology provided in step a). In other words, the hierarchical organisation of medical terms based on the graph-based ontology created in step a) ensures a certain harmonization for the sake of comparability. The resulting connected data structure or knowledge graph may be subject to clustering of data sets, to aggregation on different levels of granularity and, eventually, to analysis of a medical data record of interest or a set of medical data records of interest, e.g. by comparative assessment or trend derivation.

For the purposes of the invention a natural language processing asset (NLP) includes tools and libraries for grammatical tagging, word sense disambiguation, entity recognition, co-reference resolution, sentence parsing, word segmentation, stemming and lemmatization, etc. Additionally, the NLP includes a graph-based ontology of medical terms derived from a medical knowledge domain.

The medical knowledge domain may be any system of biomedical thesaurus(es), semantic network(s) and lexicon(s)/lexical tools. A thesaurus typically provides biomedical and health-related terms and codes including hierarchies, definitions, relationships and attributes. A semantic network typically provides categories of concepts and a set of relationships between these categories. Lexicons/lexical tools typically serve to normalize strings, generate lexical variants and create indices by means of medical and general English vocabulary. A suitable medical knowledge domain is the Unified Medical Language System (UMLS) provided and maintained by the US National Library of Medicine. The system may be customized in order to meet local, translational or specialist demands.

For the purposes of the invention, a graph-based ontology is an ontology, i.e. a collection of entity types and entity relationships, derived from the medical knowledge domain, which informs the medical terms of the medical knowledge domain and organizes them in a linked structure. The linked structure may be designed to have a desired number of hierarchy levels, e.g. 1 to 15, preferably 2 to 10, more preferably 3 to 7 hierarchy levels.

For the purposes of the invention, supervised learning means the machine learning task of learning a function that maps an input to an output based on example input-output pairs. E.g., a limited amount of labelled training datasets (example input-output pairs) is fed to the cognitive computing unit in order to train algorithms to classify data or predict outcomes accurately. Supervised learning was found particularly useful for automated classification tasks, such as e.g., document types or text segments. The human and/or machine might adjust the weight of information until a model has been fitted appropriately.

The enriching in step e) ensures, that the data elements remain comparable and suitable for cross case analysis. The association of data elements, sharing a joint parent (synonyms in a broader sense) are herein sometimes referred to as "horizontal enrichment", while the association with higher subordinate / superordinate levels of the ontology may be referred to as "vertical enrichment".

For the purposes of the invention, the clusters created in step f) may, in the simplest case, be described by a generalizing hierarchy level which combines a set of subcategories within the graph-based ontology. However, other clusters based on alternative commonalities are likewise possible and encompassed by the invention. Examples of alternative commonalities include shared initial health conditions, reoccurring complications, cases of a certain health institute or practitioner, etc.

The analysis in step g) compares and contrasts selected data elements within the knowledge graph. For example, a similarity may be computed between at least one medical data record of interest, preferably a set of medical data records of interest, and a benchmark set selected from the clustered data elements; deviations may be detected, or key figures may be calculated. For this purpose, a benchmark set selected from the data elements might need to be created. Such a benchmark set can include a set of data elements or records pertaining to specific terms, entities (cases, documents, people, dates, places, medical conditions, etc.), ontology levels, classifications, concepts, etc.

As understood in the art and according to the invention, a medical risk is a risk of an adverse outcome of a diagnostic or therapeutic procedure.

In a preferred embodiment, the method as described above includes a step
a1) retrieving a flat list of medical terms from the graph-based ontology, wherein the list contains essentially all lowest-level elements of the graph-based ontology; and
wherein the matching of the extracted and annotated data elements in step d) is targeted to the flat list of words generated in step a1). Step a1) will typically follow step a).

It is an advantage of this approach that matching has not to be done against too many terms while still being able to find as many and specific terms as reasonably possible. This reduces computational resources needed for the NLP workflow.

The method as described above may include a step
f1) displaying the clusters of step f) such that a level of aggregation is selectable by a user.

Step f1) will typically follow step f). Step f1) allows a user to visualize both the graph-based ontology and additional clusters generated in step f). The display allows a user to better locate a medical term, a specific document, a data set, a medical case, etc. within the ontology or on the basis of further concepts generated in step f). Additionally, the visualization allows a user to browse and aggregate the knowledge comprised in the data sets and matched to the graph-based ontology in a simple and intuitive way. The display in step f1) together with a suitable user interface makes it possible for a person, e.g., a chief physician, without any special programming skills to create and verify theses with respect to a medical term, specific document, data set, medical case, etc. independently.

In a preferred embodiment, the method as described above comprises a step
h) extending and/or reorganising the graph-based ontology by adding terms, classifications, and or concepts after any one of steps c), f) and g).

The graph-based ontology created in step a) is a core element of the inventive method. It not only assists in extracting and annotating the data elements of the previous medical data records, but also structures the matched data elements in step d) which in turn leads to the enrichment of data elements in step e) and the clustering in step f). Therefore, it is important that the ontology increasingly improves as the medical knowledge domain evolves and the information comprised in the previous medical data records accumulates. In order to ensure dynamic review and adaptation of the system, the ontology may be periodically regenerated. The ontology may be extended and/or reorganized by adding, redefining or reweighting terms, entities, relations, classifications, concepts, etc., wherein the modification(s) may be made manually, by supervised learning, unsupervised learning, and combinations thereof.

It is advantageous if the data elements extracted in step c) are selected from the group of document type, date, location, institution, medical staff, patient information, initial health condition, pre-existing diseases, family history, diagnosis, treatment, medication, result, complications, adverse event, root cause. In principle, any data elements that potentially provide indications of medical risk patterns are eligible. The choice of data elements to be extracted empowers a data analyst or, in case of the displaying clusters of step f) in a browsable way, an end user to formulate educated hypotheses and to detect and discover medical risks.

In a preferred embodiment of the method as described above, step c) includes:
- annotating on word level considering the a near word context, wherein the near word context includes negative/positive resolution and relation extraction; and
- annotating based on the context, considering paragraph and document classification.

It is particularly preferred that the annotation on word level is based on the NLP provided in step a) and that the annotation in a more global context is based on application of classification models trained by supervised machine learning. Typically, the training data for the supervised learning may comprise a limited set of manually classified documents / paragraphs. The extraction and annotation may be performed on a cloud platform or an SQL server.

It is preferred that the matching of the extracted and annotated data elements of step c) to the medical terms and the subsequent assignment to the graph-based ontology is performed in a recursive manner, i.e. such that each newly derived link between entities potentially enables further matching respectively further paths through the graph. With this method, fewer data sets need to be analyzed to achieve the necessary prediction accuracy, which can save time and computational resources.

It is preferred that the analyzing of a medical data record of interest in step g) is performed in a recursive manner, i.e. such that each medical risk identified in step g) potentially enables identification of further medical risks. Again, with this method, fewer data sets need to be analyzed to achieve the necessary prediction accuracy, which can save time and computational resources.

In a preferred embodiment of the method, in step g) the set of medical data records of interest and/or the benchmark set selected from the previous data records are selected based on the clustered data elements of step f). The flexible clustering based not only on superordinate ontology levels but also on independent cross-case commonalities such as shared terms or attributes enables the calculation of diverse key figures and the generation of reports, e.g. to the attention of a chief physicist. Typical patient safety issues which can be detected by the method include missed or delayed diagnosis, failure to take precautions, inadequate monitoring after treatment, failure to act on test results, non-compliance with hygiene regulations, hospital acquired infections, etc. Even major underlying reasons for materialized issues such as lack of staff training may be detected.

In an alternative embodiment, in step g) the set of medical data records of interest and/or the benchmark set selected from the previous data records are selected by a user based on at least one displayed cluster representing a level of aggregation. Due to a user-friendly data preparation and presentation, a user may compute trends and similarities, detect anomalies, and predict future developments similarly to analytical reporting but largely independently from trained data analysts.

It is advantageous if the method as described above comprises a step i) notifying a user when anomalies or medical risks surpassing a predefined threshold are detected in step h). It is an advantage of such automatic emission of an alert by the system that immanent medical risks are detected in a timely manner, bevor an adverse event occurs.

An aspect of the invention relates to a system for analyzing medical data, comprising
- one or more computers;
- a natural language processing asset (NLP), wherein the NLP includes a graph-based ontology of medical terms derived from a medical knowledge domain;
- a set of previous medical data records;
- one or more storage devices storing instructions that are operable, when executed by the one or more computers, to cause the one or more computers to perform operations comprising the method as described above.

It is particularly preferred that the system additionally comprises means for displaying the clusters generated in step
f) of the method such that a level of aggregation is selectable by a user.

The invention also relates to a non-transitory computer-readable medium storing software comprising instructions executable by one or more computers which, upon such execution, cause the one or more computers to perform operations comprising the method as described above.

The invention will be better understood based on the following examples. The examples are for illustration purposes and are not meant to limit the scope of the invention.

The figures show the following:
- Figure 1: Schematic representation of a method according to the invention;
- Figure 2: simplified overview of a method according to the invention.

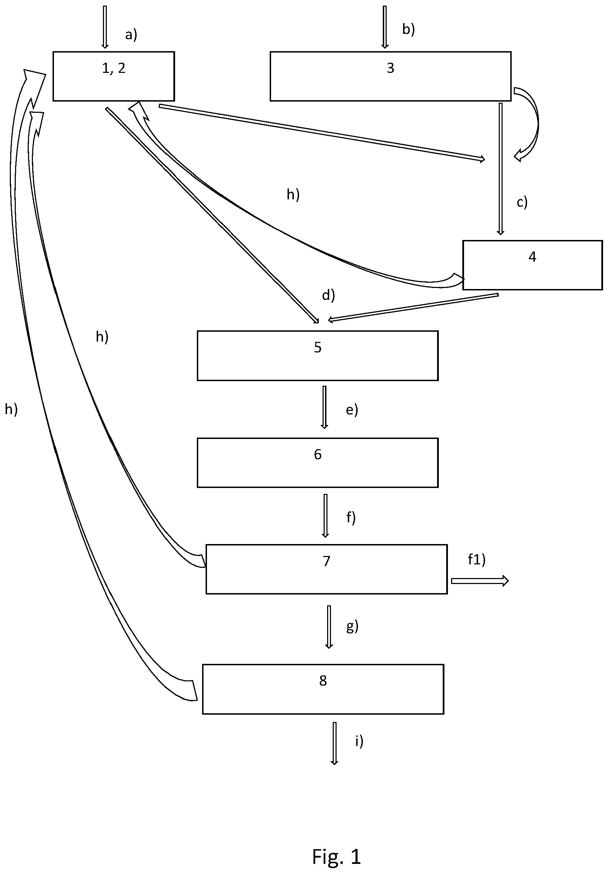

Figure 1 shows a schematic representation of the inventive method. The arrows represent method steps a) to i) and the reference numerals refer to the information used for or resulting in a respective step.

Step a) is the initial step of providing an NLP asset 1, including a graph-based ontology 2 of medical terms derived from a medical knowledge domain. Exemplarily, the medical knowledge database may be the UMLS medical database, and particularly the MedDRA Medical Dictionary for Regulatory Activities and/or the MeSH (Medical Subject Headings). The UMLS not only provides medical terms but also relations, concepts, synonyms and hierarchy levels which serve to build a graph-based ontology.

Step b) is the provision of a set of previous medical data records 3. The previous medical data records may serve to provide training data for machine learning to improve the graph-based ontology, to add data points to the knowledge graph, and to eventually improve the clustering of data elements in step f), f1). The knowledge graph is also basis for establishing the benchmark comparison in step g).

A specific example of set of previous medical data records may be all documents related to cases of obstetric emergencies upon birth which have occurred in a specific country. A specific example of terminology could be a "shoulder dystocia". The NLP may define the term as "obstetric complication during delivery in which exit of the fetus is delayed due to physical obstruction involving fetal shoulder". The term is classified as a pathologic function amongst other neonatal and maternal complications, such as hypoxia, cerebral palsy, postpartum bleeding, uterine rupture, etc. Synonyms include "shoulder girdle dystocia", "impacted shoulders", "distokie", etc. A broader concept which encompasses the term would be "foetal position and presentation abnormalities". A narrower concept could be "shoulder dystocia with antenatal problem". Medical risk factors associated with shoulder dystocia are gestational diabetes, a previous history of the condition, obesity in the mother, etc.

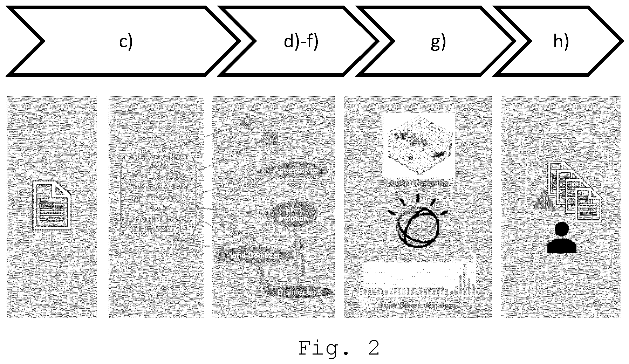

In step c) as depicted in Fig. 2, the data elements such as patient identity and medical condition, hospital of treatment, date of delivery, referring physician, family history, symptoms and treatments are extracted and tagged. As is visualized by the curved arrow involved in step c), not only the NPS 1, but also a set of previous medical cases 3 may serve as a source of classification tools.

In step d) the annotated data elements 4 are matched to the medical terms and assigned to the graph-based ontology created in step a). For example, a specific medical case could now be represented as a shoulder dystocia having occurred recently in the University Hospital of city X where a mother M diagnosed with gestational diabetes but with unobtrusive family history delivered earlier this year, and where the treatment (e.g. pushing on the abdomen above the pubic bone) by midwife N led to brachial plexus injury which in turn required surgery on a baby B at a later stage. The assigned data elements 5 form part of a linked network of elements of certain semantic types, each of them being in a relationship with at least one other element, i.e. embedded in a knowledge graph.

Once the data elements are organized, they can be enriched, e.g., by associating them to data elements sharing a joint parent, e.g. "shoulder dystocia" and "obstructed labour". This is herein referred to as horizontal enrichment. On the other hand, the data element may be associated with a more global concept, e.g "foetal position and presentation abnormalities". This is herein referred to as vertical enrichment.

In step f) the enriched data elements 6 are clustered based on concepts retrieved from the graph-based ontology 2. This can be done on a level of hierarchy (cluster of foetal position and presentation abnormalities), but also based on other, more elaborate concepts (e.g. co-occurrence of gestational diabetes and shoulder dystocia, birth related complications which required surgery).

Optionally, the clusters 7 of step f) may be displayed in a step f1) such that a level of aggregation is selectable by a user.

The clustered data elements 7, or optionally the elements resulting from step f1), may then be subject to analysis g). In particular, they can serve as a benchmark set. The analysis g) may be directed either to anomality detection (Does a particular data record of interest compare to similar medical cases in the past? How many cases of obstructed labour have occurred in hospital X as compared to other hospitals?) or it may be used for medical risk prediction (How has the number of shoulder dystocia per vaginal birth developed over time throughout the country?).

The results of the analysis 8 may be notified to a user in a step i). Especially, the user might receive an alert or warning when anomalies are detected or a medical risk surpassing a predefined threshold value is predicted in step g).

In the scheme of Figure 1, three possible routes for further developing, customizing or improving the graph-based ontology 2 are depicted by curved arrows h). Specifically, the graph-based ontology 2 may be extended or reorganised after any one of steps c), f) and g). For example, annotation of the set of previous medical data records may detect individual words of frequent occurrence and/or importance. Such words might be specific to a country or pertain to non-medical field, e.g., a legal qualification ("extraordinary case of death"). Such an entity might be worth to be included into the ontology, after step c). Further re-organizations may become necessary after step f) or g). They may be required, when data elements of very diverse/specific documents give rise to further compression/enriching steps. In such cases, the ontology might be further adapted to accommodate more concepts and/or relations.

Figure 2 shows e simplified overview of the inventive method as described above. Step c) involves the sub-steps of text pre-processing and categorization of words and context, as well as entity recognition and extraction. Steps d) to f) include the addition of semantic knowledge and the organization of terms in a linked network based on the graph-based ontology 2 as described above, in order to compress and re-enrich the data elements. In step g) similarities and/or anomalies are be computed. For example, the analysis may include time outlier detection and time series deviations. Step h), eventually is the notification of anomalies or medical risks exceeding a predefined threshold to a user, e.g. a supervising physicist, in order to prevent materialization of medical risks and to enhance patient safety.

## Claims

1. A computer implemented method for analyzing medical data, comprising the steps of
a) providing a natural language processing asset (NLP) (1), wherein the NLP (1) includes a graph-based ontology (2) of medical terms derived from a medical knowledge domain;
b) providing a set of previous medical data records (3), in particular diagnostic medical data records and/or therapeutic medical data records;
c) extracting and annotating a plurality of data elements from the set of previous medical data records, wherein the extracting and annotating is based on
- the NLP provided in step a), and/or
- classification models trained by supervised machine learning;
d) matching the extracted and annotated data elements (4) of step c) to the medical terms retrieved in step a) and assigning the matched data elements to the graph-based ontology (2);
e) enriching the matched data elements (5) of step d)
- by associating a first data element with at least a second data element sharing a parent with the first data element within the ontology; and/or
- by assigning a first data element to a subordinate or superordinate level of the ontology;
f) clustering the enriched data elements (6) of step e) based on concepts retrieved from the graph-based ontology (2);
g) analyzing a medical data record of interest or a set of medical data records of interest by
- comparing at least one medical data record of interest, preferably a set of medical data records of interest, to a benchmark set selected from the clustered data elements (7), and/or
- predicting risks associated with a medical data record of interest or a set of medical data records of interest based on classification models, preferably trained by supervised machine learning; wherein the risks are medical risks.

2. Method according to claim 1, wherein the method includes a step
a1) retrieving a flat list of medical terms from the graph-based ontology (2), wherein the list contains essentially all lowest-level elements of the graph-based ontology (2); and
wherein the matching of the extracted and annotated data elements in step d) is targeted to the flat list of words generated in step a1).

3. Method according to any of the preceding claims, wherein the medical data records provided in step b) are selected from the group of medical reports, clinical notes, discharge summaries, clinical trial protocols, laboratory test results, clinical and family history, medications, medical questionnaires, health surveys, admission reports, healthcare app data, diagnoses, radiology documents, physiologic monitoring data, clinical research datasets.

4. Method according to any one of the preceding claims, wherein the method includes a step
f1) displaying the clusters (7) of step f) such that a level of aggregation is selectable by a user.

5. Method according to any one of the preceding claims, wherein the method additionally comprises a step
h) extending and/or reorganising the graph-based ontology (2) after any one of steps c), f) and g).

6. Method according to any one of the preceding claims, wherein the data elements extracted in step c) are selected from the group of document type, date, location, institution, medical staff, patient information, initial health condition, pre-existing diseases, family history, diagnosis, treatment, medication, result, complications, adverse event, root cause.

7. Method according to any one of the preceding claims, wherein the annotation in step c) includes:
- annotating on a word level considering the a near word context, wherein the near word context includes negative/positive resolution and relation extraction; and
- annotating based on the context, considering paragraph and document classification.

8. Method according to any one of the preceding claims, wherein in step d) the matching of the extracted and annotated data elements and the subsequent assignment of the matched data elements to the graph-based ontology is performed in a recursive manner.

9. Method according to any one of the preceding claims, wherein in step g) the analyzing of a medical data record of interest is performed in a recursive manner.

10. Method according to any one of claims 4 to 9, wherein in step g) the set of medical data records of interest and/or the benchmark set selected from the previous data records are selected by a user based on at least one displayed cluster representing a level of aggregation.

11. The method of any one of the preceding claims, wherein the method comprises a step
i) notifying a user when anomalies or medical risks surpassing a predefined threshold are detected in step g).

12. A system for analyzing medical data, comprising
- one or more computers;
- a natural language processing asset (NLP), wherein the NLP includes a graph-based ontology of medical terms derived from a medical knowledge domain;
- a set of previous medical data records;
- one or more storage devices storing instructions that are operable, when executed by the one or more computers, to cause the one or more computers to perform operations comprising the method of any one of claims 1 to 11.

13. A system according to claim 12, comprising
- means for displaying the clusters generated in step f) of the method such that a level of aggregation is selectable by a user.

14. A non-transitory computer-readable medium storing software comprising instructions executable by one or more computers which, upon such execution, cause the one or more computers to perform operations comprising the method of any one of claims 1 to 11.
